# FASCICULE DE BREVET EUROPEEN

(11) **EP 3 220 985 B1**
(45) Date de publication et mention de la délivrance du brevet: **31.10.2018**
(21) Numéro de dépôt: 15817441.7
(22) Date de dépôt: 17.11.2015
(51) Int. Cl.: A61M 15/00

(54) **DISPOSITIF DE DISTRIBUTION DE PRODUIT FLUIDE DÉCLENCHÉ PAR L'INHALATION.**
INHALATIONSBETÄTIGTE ABGABEVORRICHTUNG FÜR EIN FLÜSSIGES PRODUKT.
DEVICE FOR DISPENSING A FLUID PRODUCT TRIGGERED BY INHALATION.

(30) Priorité: 21.11.2014 FR 1461291
(43) Date de publication de la demande: 27.09.2017
(73) Titulaire: APTAR France SAS, 27110 Le Neubourg (FR)
(72) Inventeur: BAILLET, Matthieu, 76000 Rouen (FR)
(74) Mandataire: CAPRI
(86) Numéro de dépôt international: PCT/FR2015/053096
(87) Numéro de publication internationale: WO 2016/079410

(56) Documents cités:
- WO-A1-98/41254
- WO-A1-99/44662
- WO-A1-2013/175121
- WO-A1-2013/178951
- GB-A- 2 461 153
- US-A- 3 157 179

## Description

La présente invention concerne un dispositif de distribution de produit fluide déclenché par l'inhalation, et plus particulièrement un dispositif du type aérosol déclenché par l'inhalation.

Les dispositifs déclenchés par l'inhalation, désignés généralement par le terme B.A.I. (signifiant "Breath Actuated Inhaler"), sont bien connus dans l'état de la technique. L'avantage principal de ce type de dispositif est que la distribution du produit est synchronisée avec l'inhalation du patient, pour garantir une bonne distribution du produit. Ainsi, dans le domaine des dispositifs aérosol, c'est-à-dire ceux dans lesquels le produit est distribué à l'aide d'un gaz propulseur, de nombreux types de dispositifs de déclenchement par l'inhalation ont été proposés. Ces dispositifs présentent toutefois l'inconvénient de comporter un grand nombre de pièces, c'est-à-dire qu'ils sont compliqués et coûteux à fabriquer et à assembler, ce qui est évidemment désavantageux. Il est aussi difficile de trouver le bon équilibre entre un déclenchement fiable à chaque inhalation, sans que le seuil de déclenchement ne soit trop élevé, et une serrure suffisamment robuste pour empêcher un actionnement accidentel ou non souhaité.

Les documents US3157179, WO9944662 et GB2461153 décrivent des dispositifs de ce type de l'état de la technique.

La présente invention a pour but de fournir un dispositif d'inhalation déclenché par l'inhalation qui ne reproduit pas les inconvénients susmentionnés.

La présente invention a aussi pour but de fournir un dispositif d'inhalation déclenché par l'inhalation qui améliore la fiabilité de fonctionnement, en garantissant un actionnement à chaque inhalation.

La présente invention a aussi pour but de fournir un dispositif d'inhalation déclenché par l'inhalation qui minimise les risques d'actionnement accidentel ou non souhaité.

La présente invention a également pour but de fournir un dispositif d'inhalation déclenché par l'inhalation qui ne présente pas un seuil de déclenchement trop élevé, permettant ainsi à des personnes relativement faibles, telles que des personnes malades ou âgées, d'utiliser le dispositif de manière sûre et fiable.

La présente invention a également pour but de fournir un dispositif d'inhalation déclenché par l'inhalation qui soit simple et peu coûteux à fabriquer et à assembler.

La présente a donc pour objet un dispositif de distribution de produit fluide déclenché par l'inhalation, comportant un réservoir de produit contenant un produit fluide et un gaz propulseur, une valve doseuse étant assemblée sur ledit réservoir pour distribuer sélectivement le produit fluide, ledit dispositif comportant :
- un organe d'armement déplaçable d'une part entre une position de repos et une position chargée en comprimant un ressort, et déplaçable d'autre part axialement entre ladite position chargée et une position de distribution sous l'effet de la force exercée par ledit ressort comprimé,
- des moyens de blocage pour bloquer ledit organe d'armement dans ladite position chargée,
- un système de déclenchement commandé par l'inhalation pour libérer lesdits moyens de blocage,
ledit organe d'armement comportant une denture et le dispositif comportant en outre:
- un élément de support de réservoir fixé audit réservoir et comportant une ouverture de came, et
- une roue d'engrènement comportant d'une part une projection dentée, adaptée à coopérer avec ladite denture dudit organe d'armement, et d'autre part une projection de came, adaptée à coopérer avec ladite ouverture de came dudit élément de support de réservoir, ladite projection dentée étant sensiblement centrée par rapport à l'axe de rotation de ladite roue d'engrènement et ladite projection de came étant désaxée par rapport audit axe de rotation, ladite roue d'engrènement coopérant avec ledit organe d'armement de telle sorte que lorsque ledit organe d'armement se déplace de sa position chargée vers sa position de distribution, ladite roue d'engrènement fait un tour complet, de telle sorte que ladite projection de came effectue un déplacement circulaire provoquant le déplacement axial en va-et-vient dudit élément de support de réservoir, avec un premier déplacement axial pour actionner ladite valve et un second déplacement axial en sens inverse pour ramener la valve en position de repos.

Avantageusement, le dispositif comporte un organe d'actionnement coopérant avec ledit organe d'armement, ledit organe d'actionnement étant pourvu d'une surface de came pour déplacer ledit organe d'armement d'une position de repos vers une position chargée, ledit organe d'actionnement coopérant dans ladite position chargée avec lesdits moyens de blocage pour maintenir ledit organe d'armement dans ladite position chargée jusqu'à l'inhalation, et ledit organe d'actionnement étant déplacé par ledit organe d'armement vers une position de distribution après libération desdits moyens de blocage pour actionner ladite valve.

Avantageusement, lesdits moyens de blocage comportent un verrou coopérant en position de blocage avec une projection dudit organe d'actionnement.

Avantageusement, ledit système de déclenchement commandé par l'inhalation comporte une chambre d'air déformable qui est déformée lors de l'inhalation, ladite chambre d'air déformable étant fixée à un élément déclencheur, tel qu'une tige pivotante.

Avantageusement, lesdits moyens de blocage sont solidaires dudit élément déclencheur.

Avantageusement, ledit élément de support de réservoir coopère avec une plaque de support solidaire dudit corps.

Avantageusement, ladite plaque de support comporte des éléments de maintien, adaptés à coopérer avec des rampes de maintien dudit élément de support de réservoir.

Avantageusement, ladite plaque de support comporte des nervures de guidage rectilignes, adaptées à coopérer avec des pions de guidage dudit élément de support de réservoir.

Ces caractéristiques et avantages et d'autres apparaîtront plus clairement au cours de la description détaillée suivante, faite en référence aux dessins joints, donnés à titre d'exemples non limitatifs, et sur lesquels :
- les figures 1a et 1b sont deux vues schématiques partiellement découpées d'un dispositif d'inhalation déclenché par l'inhalation, selon un mode de réalisation avantageux de la présente invention, en position de repos,
- la figure 2 est une vue de détail de la figure 1b illustrant la serrure de verrouillage dans ladite position de repos,
- la figure 3 est une vue de détail de la figure 1b illustrant le système d'armement dans ladite position de repos,
- les figures 4a et 4b sont deux vues similaires à celles des figures 1a et 1b, en position armée et non déclenchée,
- la figure 5 est une vue similaire à celle de la figure 2, dans ladite position armée et non déclenchée,
- la figure 6 est une vue similaire à celle de la figure 3, dans ladite position armée et non déclenchée,
- la figure 7 est une vue de détail de la roue d'engrènement, dans ladite position armée et non déclenchée,
- la figure 8 est une vue de détail du pion de la roue d'engrènement dans la came du support de réservoir, dans ladite position armée et non déclenchée,
- les figures 9a et 9b sont deux vues similaires à celles des figures 4a et 4b, en position déclenchée, après un quart de la course axiale du coulisseau,
- la figure 10 est une vue similaire à celle de la figure 7, dans ladite position déclenchée, après un quart de la course axiale du coulisseau,
- la figure 11 est une vue similaire à celle de la figure 8, dans ladite position déclenchée, après un quart de la course axiale du coulisseau,
- les figures 12a et 12b sont deux vues similaires à celles des figures 9a et 9b, en position déclenchée, après la moitié de la course axiale du coulisseau,
- la figure 13 est une vue similaire à celle de la figure 5, dans ladite position déclenchée, après la moitié de la course axiale du coulisseau,
- la figure 14 est une vue similaire à celle de la figure 6, dans ladite position déclenchée, après la moitié de la course axiale du coulisseau,
- la figure 15 est une vue similaire à celle de la figure 10, dans ladite position déclenchée, après la moitié de la course axiale du coulisseau,
- la figure 16 est une vue similaire à celle de la figure 11, dans ladite position déclenchée, après la moitié de la course axiale du coulisseau,
- les figures 17a et 17b sont deux vues similaires à celles des figures 12a et 12b, en position déclenchée, après trois-quarts de la course axiale du coulisseau,
- la figure 18 est une vue similaire à celle de la figure 15, dans ladite position déclenchée, après trois-quarts de la course axiale du coulisseau,
- la figure 19 est une vue similaire à celle de la figure 16, dans ladite position déclenchée, après trois-quarts de la course axiale du coulisseau,
- les figures 20a et 20b sont deux vues similaires à celles des figures 17a et 17b, en position déclenchée, après la course axiale complète du coulisseau,
- la figure 21 est une vue similaire à celle de la figure 13, dans ladite position déclenchée, après la course axiale complète du coulisseau,
- la figure 22 est une vue similaire à celle de la figure 14, dans ladite position déclenchée, après la course axiale complète du coulisseau,
- la figure 23 est une vue similaire à celle de la figure 18, dans ladite position déclenchée, après la course axiale complète du coulisseau,
- la figure 24 est une vue similaire à celle de la figure 19, dans ladite position déclenchée, après la course axiale complète du coulisseau,
- la figure 25 est une vue schématique de côté de la roue d'engrènement, selon un mode de réalisation avantageux de la présente invention,
- la figure 26 est une vue schématique de dessus de la roue d'engrènement de la figure 25,
- la figure 27 est une vue schématique de dessus du support de réservoir, selon un mode de réalisation avantageux de la présente invention,
- la figure 28 est une vue schématique de dessous du support de réservoir de la figure 27, et
- la figure 29 est une vue schématique de dessus de la plaque de guidage, selon un mode de réalisation avantageux de la présente invention.

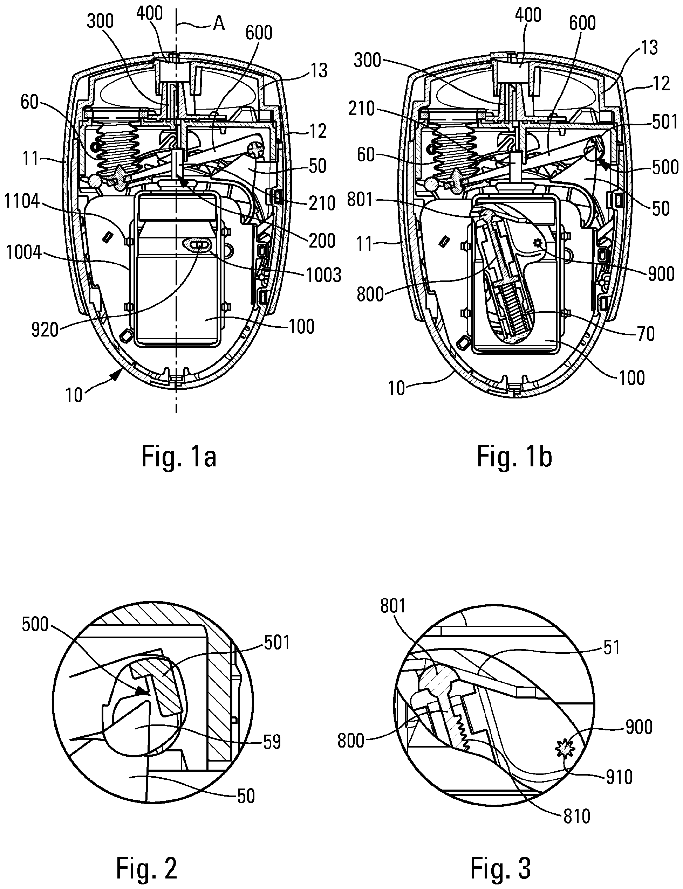

Dans la description, les termes "haut" et "bas" se réfèrent à la position représentée notamment sur la figure 1a. Le terme "axial" se réfère à l'axe central vertical A représenté sur la figure 1a.

L'invention s'applique plus particulièrement à des dispositifs d'inhalation du type à valve aérosol pour une distribution orale, comme cela sera décrit plus en détail ci-après, mais elle pourrait aussi s'appliquer à d'autres types de dispositifs d'inhalation, par exemple du type nasal.

Les figures représentent un mode de réalisation avantageux de l'invention, mais il est entendu l'une ou plusieurs des pièces constitutives décrites ci-après pourrai(en)t être réalisée(s) différemment tout en procurant des fonctions similaires voire identiques.

En référence aux dessins, le dispositif d'inhalation comporte un corps principal 10 sur lequel peuvent être montés coulissants deux éléments de capot 11, 12, adaptés à être ouverts pour ouvrir et charger le dispositif. Le corps principal 10 peut être de forme environ arrondie comme représenté sur les figures, mais il pourrait avoir toute autre forme appropriée. Un corps supérieur 13 est assemblé au corps principal 10, formant un embout buccal 400. Cet embout buccal 400 définit un orifice de distribution à travers lequel l'utilisateur va inhaler lors de l'actionnement du dispositif. Cet orifice de distribution est typiquement disposé environ au centre de l'embout buccal 400. Les éléments de capot 11, 12 peuvent s'ouvrir par pivotement autour d'un axe de rotation commun, ou autour de deux axes parallèles en étant engrené l'un avec l'autre. Tout autre moyen d'ouverture du dispositif est envisageable. En variante, le dispositif pourrait ne comporter qu'un seul élément capot au lieu de deux.

Le corps 10 contient un réservoir 100, contenant le produit à distribuer et un gaz propulseur, tel qu'un gaz du type HFA, une valve doseuse 200 étant montée sur ledit réservoir 100 pour distribuer sélectivement le produit. La valve doseuse 200 comporte une soupape 210 axialement déplaçable par rapport audit réservoir 100 lors de l'actionnement. Cette valve doseuse 200 peut être d'un type quelconque approprié. Avantageusement, la soupape 210 est fixe par rapport au corps 10, et c'est le réservoir 100 qui est déplacé axialement par rapport au corps 10 lors de l'actionnement. L'orifice de sortie de la soupape 210 de ladite valve doseuse 200 est relié via un canal 300 à l'orifice de distribution de l'embout buccal 400, à travers lequel l'utilisateur inhale le produit distribué. Cet orifice de distribution peut bien entendu être réalisé d'une manière quelconque en fonction de l'application souhaitée du dispositif. La présente description sera faite en référence à un inhalateur comportant un embout buccal 400, mais il est clair que l'invention s'applique également à d'autres types d'utilisations, tels que par exemple un inhalateur nasal.

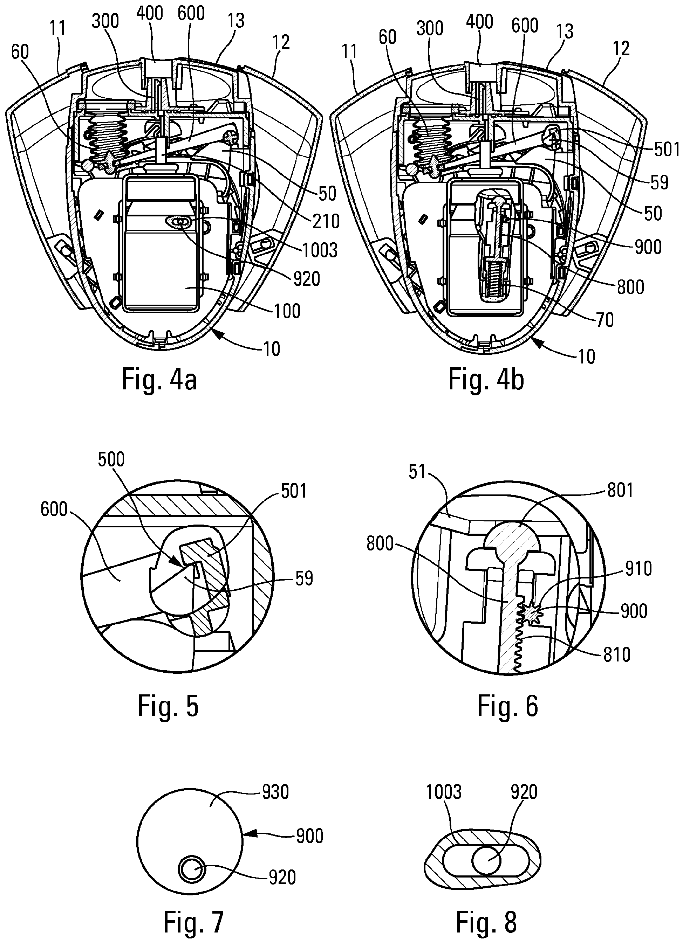

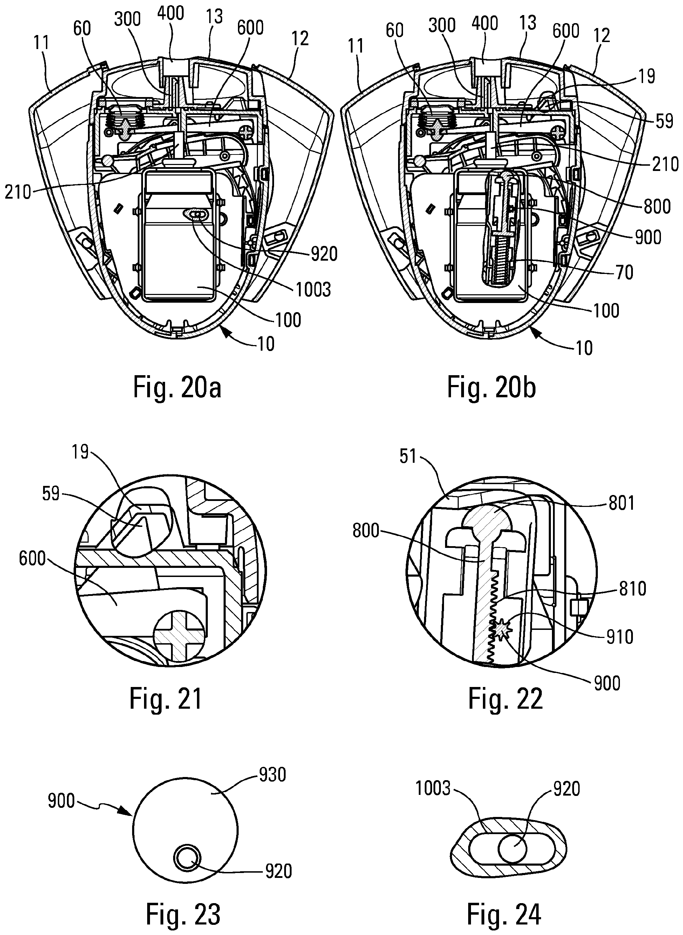

Le dispositif comporte un système de chargement. Ce système de chargement comporte un organe d'armement 800. Cet organe d'armement 800 est solidaire d'au moins un des éléments de capot 11, 12. Cet organe d'armement 800 peut se déplacer contre un ressort 70, avantageusement un ressort à boudins, d'abord entre une position de repos, représentée sur la figure 3, et une position chargée, représentée sur la figure 8, et ensuite entre ladite position chargée et une position de distribution dans laquelle il a terminé sa course d'actionnement. Les figures 20a, 20b, 21, 22, 23 et 24 illustrent cette position de distribution. L'organe d'armement 800 est connecté d'un côté audit ressort 70 et de l'autre côté, il coopère avec un organe d'actionnement 50 monté déplaçable, notamment pivotant, sur le corps 10, d'abord entre une position de repos et une position chargée, puis entre ladite position chargée et une position de distribution. La position de repos, la position chargée et la position de distribution de l'organe d'actionnement 50 correspondent respectivement à la position de repos, la position chargée et la position de distribution de l'organe d'armement 800.

L'organe d'armement 800 comporte une denture 810, s'étendant de préférence axialement sur un côté dudit organe d'armement 800.

Lorsque les éléments de capot 11, 12 sont ouverts, l'organe d'armement 800 est déplacé de sa position de repos vers sa position chargée, en comprimant le ressort 70. Lors de ce déplacement vers sa position chargée, l'organe d'armement 800 est non seulement déplacé le long de son axe longitudinal pour comprimer ledit ressort 70, mais il pivote également par rapport au corps 10, comme illustré sur les figures 1b et 4b. Simultanément, l'organe d'actionnement 50 est également déplacé de sa position de repos vers sa position chargée. En position chargée, l'organe d'actionnement 50 est empêché de se déplacer par des moyens de blocage 500, qui ne seront libérés qu'au moment de l'inhalation. Ainsi, en l'absence d'inhalation dans la position ouverte, la fermeture des éléments de capot 11, 12 provoque simplement le retour à la position de repos pour l'organe d'armement 800 et l'organe d'actionnement 50, et la décompression du ressort 70. Il n'y a donc aucun risque qu'une dose de produit actif soit perdue par un actionnement malencontreux ou incomplet dans lequel l'utilisateur n'exercerait pas d'inhalation entre l'ouverture et la fermeture des éléments de capot 11, 12. L'actionnement de la valve 200 et l'expulsion d'une dose de produit fluide ne sont donc possibles que si l'utilisateur inhale.

Les moyens de blocage 500 comportent un verrou 501 en forme de coin adapté à recevoir en position de blocage une projection 59 de l'organe d'actionnement 50. Ce verrou 501 est avantageusement solidaire, notamment monobloc, avec un élément déclencheur 600. Cet élément déclencheur 600, par exemple réalisé sous la forme d'une tige, est mobile, notamment pivotant, par rapport au corps 10. Le déplacement ou pivotement dudit élément déclencheur 600 permet ainsi de libérer lesdits moyens de blocage 500, en déplaçant ledit verrou 501 par rapport à ladite projection 59 de l'organe d'actionnement 50. De préférence, en position de repos, lesdits moyens de blocage ne sont pas en contact dudit organe d'actionnement 50, comme visible sur la figure 2. Ce n'est qu'en position chargée, visible sur la figure 5, que le verrou 501 des moyens de blocage 500 coopère avec ledit organe d'actionnement 50 pour le bloquer et empêcher l'actionnement de la valve 200.

Une surface de came 51 est formée sur ledit organe d'actionnement 50, sur laquelle glisse l'organe d'armement 800. Avantageusement, l'organe d'armement 800 comporte, dans sa partie en contact avec la surface de came 51, une partie arrondie 801 pour favoriser le glissement de l'organe d'armement 800 sur ladite surface de came 51. La surface de came 51 comporte au moins deux parties de came avec des pentes différentes. Une première partie de chargement, typiquement inclinée par rapport à l'axe A de la figure 1a, est prévue pour déplacer l'organe d'armement 800 vers sa position chargée en comprimant le ressort 70. Une seconde partie d'extrémité, typiquement environ transversale audit axe A, est prévue pour maintenir l'organe d'armement 800 en position chargée et pour coopérer avec lui lorsqu'il se déplace de sa position chargée vers sa position de distribution.

En position de distribution, l'organe d'actionnement 50 a donc été déplacé vers le haut (dans l'orientation des figures) par l'organe d'armement 800 sous l'effet dudit ressort comprimé 70.

Le dispositif comporte un système de déclenchement commandé par l'inhalation de l'utilisateur, qui est destiné à actionner la valve doseuse 200 lorsque l'utilisateur inhale à travers l'embout buccal 400.

Ce système comporte une chambre d'air 60 déformable sous l'effet de l'inhalation, cette chambre d'air 60 étant adaptée à libérer lesdits moyens de blocage 500. L'inhalation de l'utilisateur provoque la déformation de ladite chambre d'air déformable 60, permettant ainsi de libérer lesdits moyens de blocage 500 et donc l'actionnement de la valve 200.

Avantageusement, cette chambre d'air 60 comprend une membrane déformable, tel qu'un soufflet, une poche ou un diaphragme, qui peut être reliée d'une part à l'embout buccal 400 et d'autre part auxdits moyens de blocage 500 de manière directe ou indirecte. Ainsi, lors de l'inhalation, la chambre d'air 60 se déforme et/ou se contracte provoquant le déplacement desdits moyens de blocage 500 dans une position de déblocage.

La chambre d'air déformable 60 est avantageusement connectée audit élément déclencheur 600 qui supporte les moyens de blocage 500, qui bloquent l'organe d'actionnement 50. Ainsi, lors de l'inhalation de l'utilisateur, ladite chambre d'air déformable 60 se déforme, faisant pivoter l'élément déclencheur 600, et libérant ainsi lesdits moyens de blocage 500. Ceci permet le déplacement dudit organe d'actionnement 50 vers sa position de distribution sous l'effet de la force exercée par le ressort comprimé 70 sur l'organe d'armement 800. Ce déplacement provoque l'actionnement de la valve 200 et la distribution d'une dose.

La valve 200 n'est donc actionnée qu'au moment de l'inhalation, de sorte que la dose de produit fluide est expulsée hors de l'orifice de distribution simultanément à l'inhalation.

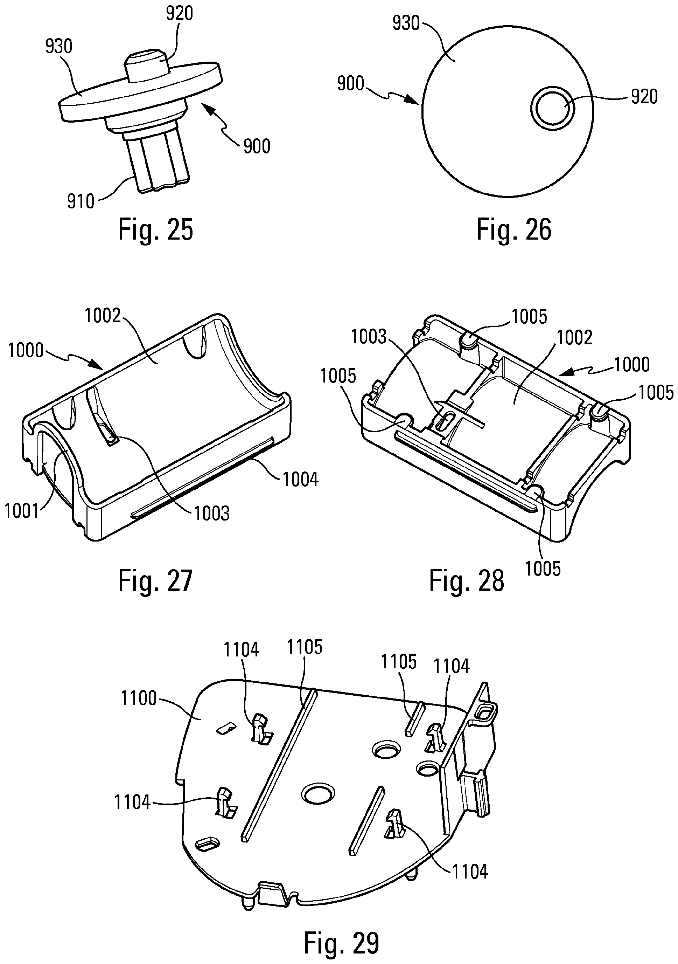

Le dispositif comporte une roue d'engrènement 900, visible notamment sur les figures 25 et 26. Cette roue d'engrènement 900 comporte d'une part une projection dentée 910, adaptée à coopérer avec ladite denture 810 dudit organe d'armement 800, et d'autre part une projection de came 920, adaptée à coopérer avec ledit réservoir 100 ou une partie solidaire de celui-ci. La projection dentée 910 est sensiblement centrée par rapport à l'axe de rotation de ladite roue d'engrènement 900 alors que la projection de came 920 est désaxée par rapport à cet axe de rotation, tout en s'étendant dans une direction parallèle à celle de ladite projection dentée 910. Comme visible sur la figure 25, la projection dentée 910 s'étend d'un côté d'un disque 930 alors que ladite projection de came 920 s'étend de l'autre côté dudit disque 930. La denture de la projection dentée 910 et la denture 810 de l'organe d'armement 800 sont réalisées de telle sorte qu'une course axiale d'actionnement complète dudit organe d'armement 800 correspond à un tour complet de ladite roue d'engrènement 900.

Le dispositif comporte en outre un élément de support de réservoir 1000 dans lequel est fixé le réservoir 100. Cet élément de support 1000 peut comporter une paroi de fond 1001 et une paroi semi-cylindrique 1002 pour recevoir et maintenir ledit réservoir 100, comme visible notamment sur les figures 27 et 28. La paroi semi-cylindrique 1002 comporte une ouverture de came 1003, de forme oblongue ou allongée, ainsi que des rampes de maintien 1004 et des pions de guidage 1005 de chaque coté de ladite paroi semi-cylindrique 1002. Ladite ouverture de came 1003 reçoit ladite projection de came 920 de ladite roue d'engrènement 900.

Avantageusement, le dispositif comporte également une plaque de support 1100 solidaire du corps 10, c'est-à-dire non mobile par rapport à celui-ci. Cette plaque de support 1100 coopère avec ledit élément de support de réservoir 1000, ce dernier étant déplaçable en translation par rapport à ladite plaque de support 1100. Comme visible sur la figure 29, ladite plaque de support 1100 comporte des éléments de maintien 1104, adaptés à coopérer avec lesdites rampes de maintien 1004 dudit élément de support de réservoir 1000, ainsi que des nervures de guidage 1105 rectilignes, adaptées à coopérer avec lesdits pions de guidage 1005 dudit élément de support de réservoir 1000.

Lorsque l'utilisateur souhaite utiliser le dispositif, il ouvre les éléments de capot 11, 12, ce qui a pour effet d'actionner le système de chargement. L'organe d'armement 800 est déplacé vers sa position chargée ce qui a pour effet de comprimer le ressort 70 qui exerce alors une force axiale sur l'organe d'actionnement 50 pour solliciter ce dernier vers sa position de distribution. Pendant ce déplacement de l'organe d'armement 800 vers sa position chargée, l'organe d'actionnement 50 est déplacé pour venir en contact avec le verrou 501 des moyens de blocage 500, de sorte qu'en position chargée, l'organe d'actionnement 50 est retenu et bloqué contre tout déplacement vers sa position de distribution. Simultanément, la denture 810 de l'organe d'armement 800 s'engrène dans la projection dentée 910 de ladite roue d'engrènement 900. Ainsi, pendant cette phase de chargement, il n'y a pas d'action sur le réservoir 100, qui reste immobile par rapport au corps 10. Dans cette position chargée, représentée sur les figures 4a, 4b, 5, 6, 7 et 8, le dispositif est donc en position d'attente.

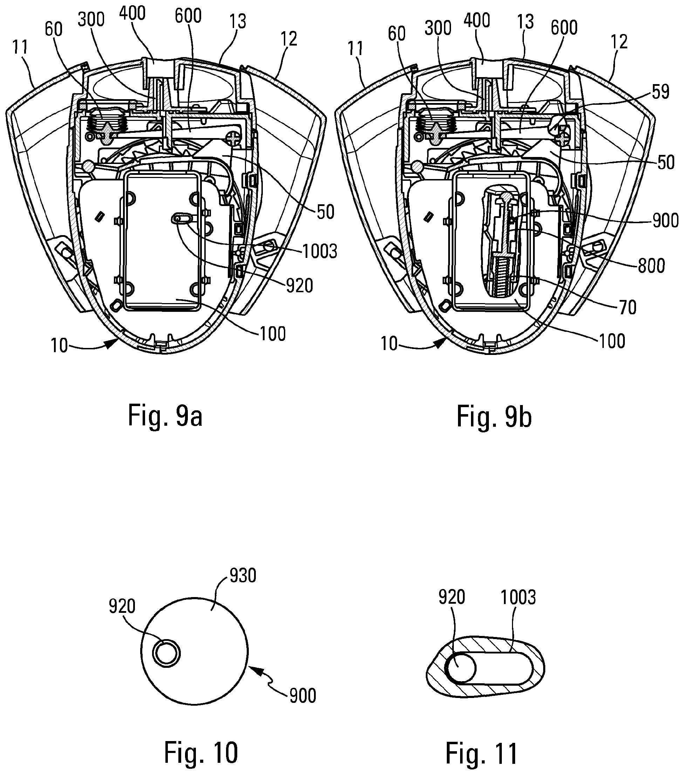

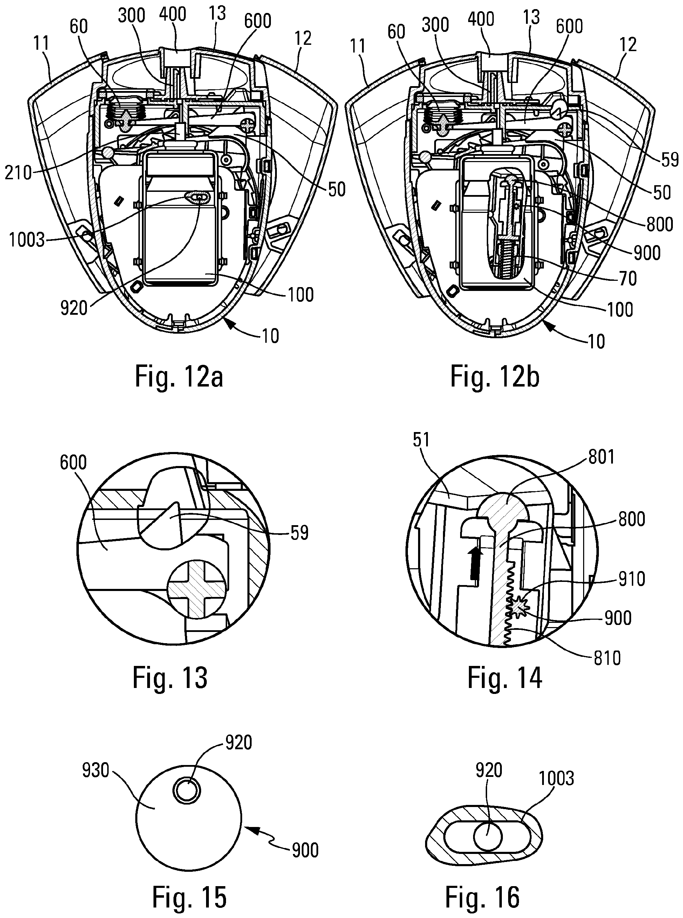

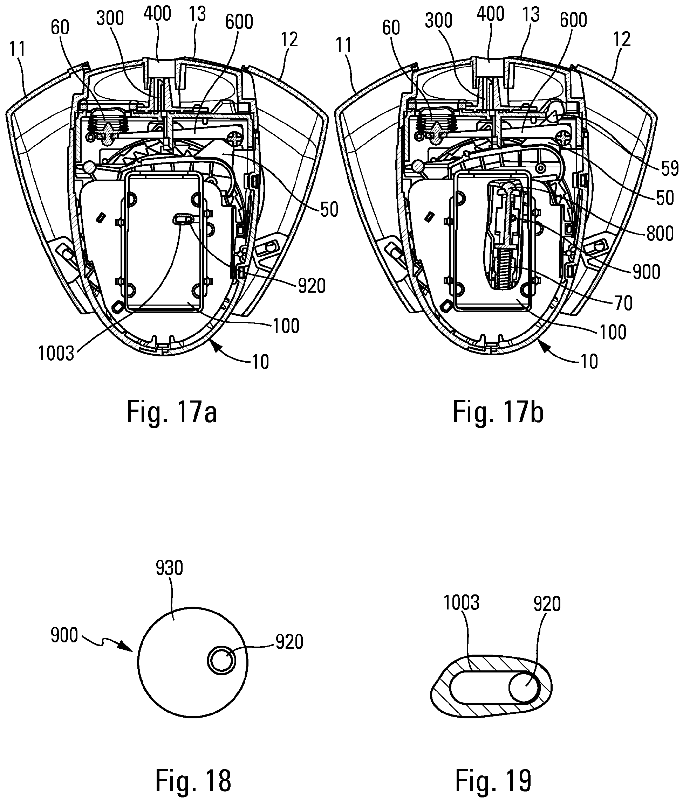

Lorsque l'utilisateur inhale à travers l'embout buccal 400, il va déformer la chambre d'air déformable 60, ce qui va faire pivoter l'élément déclencheur 600 fixé à ladite chambre d'air 60. Ce déplacement de l'élément déclencheur 600 va faire pivoter le verrou 501 par rapport à la projection 59 de l'organe d'actionnement 50, pour libérer les moyens de blocage 500. Sous l'effet du ressort 70, l'organe d'armement 800 va donc être déplacé axialement vers le haut et ainsi pousser l'organe d'actionnement 50 axialement vers le haut, comme visible sur les figures 9a à 24.

Ce déplacement axial de l'organe d'armement 800 va faire tourner la roue d'engrènement 900. La projection de came 920 de ladite roue d'engrènement 900 étant désaxée par rapport à l'axe de rotation, en particulier disposée à proximité de la périphérie dudit disque 930, elle va effectuer un mouvement circulaire lors de la rotation de ladite roue d'engrènement. Les figures 7, 10, 15, 18 et 23 illustrent en détail la position de ladite projection de came 920 respectivement au début de la rotation, après un quart de tour, après un demi-tour, après trois-quarts de tour et après un tour complet de ladite roue d'engrènement 900.

La projection de came 920 étant disposée dans l'ouverture de came 1003 dudit élément de support de réservoir 1000, ce mouvement circulaire de la projection de came 920 va d'une part déplacer celle-ci latéralement dans ladite ouverture de came 1003, comme illustré en détail sur les figures 8, 11, 16, 19 et 24, mais elle va également entrainer ledit élément de support de réservoir 1000 en déplacement axial par rapport au corps 10 et donc par rapport à la soupape 210.

Ledit élément de support de réservoir 1000 ne peut se déplacer par rapport à ladite plaque de support 1100 qu'en translation, de par la coopération des pions de guidage 1005 avec les nervures de guidage 1105. La coopération entre les éléments de maintien 1104 et les rampes de maintien 1004 assure aussi une parfaite translation dudit élément de support de réservoir 1000 par rapport à ladite plaque de support 1100. Par conséquent, sur la première moitié de la course axiale de l'organe d'armement 800, c'est-à-dire sur le premier demi-tour de la roue d'engrènement 900, la projection de came 920 déplace ledit élément de support de réservoir 1000, et donc ledit réservoir 100, axialement vers le haut, ce qui va actionner la valve 200. Sur la seconde moitié de la course axiale de l'organe d'armement 800, c'est-à-dire sur le second demi-tour de la roue d'engrènement 900, la projection de came 920 va revenir vers sa position de départ et donc déplacer ledit élément de support de réservoir 1000 ensemble avec ledit réservoir 100, axialement vers le bas, ce qui va ramener la valve dans sa position de repos.

Ainsi une course complète de l'organe d'armement 800, et donc un tour complet de la roue d'engrènement 900, correspond à un cycle complet d'actionnement de la valve 200, avec un déplacement relatif en va-et-vient entre le réservoir 100 et la soupape 210 de la valve 200.

La position de distribution de l'organe d'armement 800, c'est-à-dire la fin de sa course axiale d'actionnement, est définie par une butée entre ladite projection 59 de l'organe d'actionnement 50 et une partie fixe 19 par rapport au corps 10, comme visible sur la figure 21

En fin d'inhalation, l'utilisateur referme les éléments de capot 11, 12, ce qui ramène le dispositif en position de repos, en étant prêt pour une utilisation ultérieure.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide déclenché par l'inhalation, comportant un réservoir de produit (100) contenant un produit fluide et un gaz propulseur, une valve doseuse (200) étant assemblée sur ledit réservoir (100) pour distribuer sélectivement le produit fluide, ledit dispositif comportant :
- un organe d'armement (800) déplaçable d'une part entre une position de repos et une position chargée en comprimant un ressort (70), et déplaçable d'autre part axialement entre ladite position chargée et une position de distribution sous l'effet de la force exercée par ledit ressort comprimé,
- des moyens de blocage (500) pour bloquer ledit organe d'armement dans ladite position chargée,
- un système de déclenchement commandé par l'inhalation pour libérer lesdits moyens de blocage (500),
**caractérisé en ce que** ledit organe d'armement (800) comporte une denture (810) et **en ce que** le dispositif comporte en outre:
- un élément de support de réservoir (1000) fixé audit réservoir (100) et comportant une ouverture de came (1003), et
- une roue d'engrènement (900) comportant d'une part une projection dentée (910), adaptée à coopérer avec ladite denture (810) dudit organe d'armement (800), et d'autre part une projection de came (920), adaptée à coopérer avec ladite ouverture de came (1003) dudit élément de support de réservoir (1000), ladite projection dentée (910) étant sensiblement centrée par rapport à l'axe de rotation de ladite roue d'engrènement (900) et ladite projection de came (920) étant désaxée par rapport audit axe de rotation, ladite roue d'engrènement (900) coopérant avec ledit organe d'armement (800) de telle sorte que lorsque ledit organe d'armement (800) se déplace de sa position chargée vers sa position de distribution, ladite roue d'engrènement (900) fait un tour complet, de telle sorte que ladite projection de came (920) effectue un déplacement circulaire provoquant le déplacement axial en va-et-vient dudit élément de support de réservoir (1000), avec un premier déplacement axial pour actionner ladite valve (200) et un second déplacement axial en sens inverse pour ramener la valve en position de repos.

2. Dispositif selon la revendication 1, comportant un organe d'actionnement (50) coopérant avec ledit organe d'armement (800), ledit organe d'actionnement (50) étant pourvu d'une surface de came (51) pour déplacer ledit organe d'armement (800) d'une position de repos vers une position chargée, ledit organe d'actionnement (50) coopérant dans ladite position chargée avec lesdits moyens de blocage (500) pour maintenir ledit organe d'armement (800) dans ladite position chargée jusqu'à l'inhalation, et ledit organe d'actionnement (50) étant déplacé par ledit organe d'armement (800) vers une position de distribution après libération desdits moyens de blocage (500) pour actionner ladite valve (200).

3. Dispositif selon la revendication 2, dans lequel lesdits moyens de blocage (500) comportent un verrou (501) coopérant en position de blocage avec une projection (59) dudit organe d'actionnement (50).

4. Dispositif selon l'une quelconque des revendications précédentes, dans lequel ledit système de déclenchement commandé par l'inhalation comporte une chambre d'air déformable (60) qui est déformée lors de l'inhalation, ladite chambre d'air déformable (60) étant fixée à un élément déclencheur (600), tel qu'une tige pivotante.

5. Dispositif selon la revendication 4, dans lequel lesdits moyens de blocage (500) sont solidaires dudit élément déclencheur (600).

6. Dispositif selon l'une quelconque des revendications précédentes, dans lequel ledit élément de support de réservoir (1000) coopère avec une plaque de support (1100) solidaire dudit corps (10).

7. Dispositif selon la revendication 6, dans lequel ladite plaque de support (1100) comporte des éléments de maintien (1104), adaptés à coopérer avec des rampes de maintien (1004) dudit élément de support de réservoir (1000).

8. Dispositif selon la revendication 6 ou 7, dans lequel ladite plaque de support (1100) comporte des nervures de guidage (1105) rectilignes, adaptées à coopérer avec des pions de guidage (1005) dudit élément de support de réservoir (1000).

## Patentansprüche

1. Ausgabevorrichtung für ein fluides Produkt, die durch Inhalation ausgelöst wird, umfassend einen Produktbehälter (100), der ein fluides Produkt und ein Treibgas enthält, ein Dosierventil (200), das auf dem Behälter (100) montiert ist, um das fluide Produkt selektiv auszugeben, wobei die Vorrichtung umfasst:
- eine Spanneinrichtung (800), die einerseits zwischen einer Ruheposition und einer belasteten Position unter Zusammendrücken einer Feder (70) verschiebbar ist, und die andererseits unter der Wirkung der durch die zusammengedrückte Feder ausgeübten Kraft axial zwischen der belasteten Position und einer Ausgabeposition verschiebbar ist,
- Sperrmittel (500) zum Sperren der Spanneinrichtung in der belasteten Position,
- ein Auslösesystem, das durch Inhalation gesteuert wird, um die Sperrmittel (500) freizusetzen,
**dadurch gekennzeichnet, dass** die Spanneinrichtung (800) eine Verzahnung (810) umfasst und dass die Vorrichtung des Weiteren umfasst:
- ein Behälterträgerelement (1000), das an dem Behälter (100) befestigt ist und eine Nockenöffnung (1003) umfasst, und
- ein Zahnrad (900), das einerseits einen gezahnten Vorsprung (910) umfasst, der dazu geeignet ist, mit der Verzahnung (810) der Spanneinrichtung (800) zusammenzuwirken, und andererseits einen Nockenvorsprung (920) umfasst, der dazu geeignet ist, mit der Nockenöffnung (1003) des Behälterträgerelements (1000) zusammenzuwirken, wobei der gezahnte Vorsprung (910) in Bezug auf die Drehachse des Zahnrads (900) und den Nockenvorsprung (920), der in Bezug auf die Drehachse versetzt ist, im Wesentlichen zentriert ist, wobei das Zahnrad (900) derart mit der Spanneinrichtung (800) zusammenwirkt, dass, wenn sich die Spanneinrichtung (800) von ihrer belasteten Position in ihre Ausgabeposition verschiebt, das Zahnrad (900) eine komplette Umdrehung ausführt, so dass der Nockenvorsprung (920) eine kreisförmige Verschiebung bewirkt, die die axiale Verschiebung des Behälterträgerelements (1000) hin- und her bewirkt, mit einer ersten axialen Verschiebung, um das Ventil (200) zu betätigen, und einer zweiten axialen Verschiebung in umgekehrte Richtung, um das Ventil in seine Ruheposition zurückzuführen.

2. Vorrichtung nach Anspruch 1, umfassend eine Betätigungseinrichtung (50), die mit der Spanneinrichtung (800) zusammenwirkt, wobei die Betätigungseinrichtung (50) mit einer Nockenfläche (51) versehen ist, um die Spanneinrichtung (800) von einer Ruheposition in eine belastete Position zu verschieben, wobei die Betätigungseinrichtung (50) in der belasteten Position mit den Sperrmitteln (500) zusammenwirkt, um die Spanneinrichtung (800) bis zur Inhalation in der belasteten Position zu halten, und die Betätigungseinrichtung (50) durch die Spanneinrichtung (800) nach Freisetzung der Sperrmittel (500) in eine Ausgabeposition verschoben wird, um das Ventil (200) zu betätigen.

3. Vorrichtung nach Anspruch 2, wobei die Sperrmittel (500) einen Bolzen (501) umfassen, der in der Sperrposition mit einem Vorsprung (59) der Betätigungseinrichtung (50) zusammenwirkt.

4. Vorrichtung nach einem der vorhergehenden Ansprüche, wobei das Auslösesystem, das durch Inhalation gesteuert wird, eine verformbare Luftkammer (60) umfasst, die bei Inhalation verformt wird, wobei die verformbare Luftkammer (60) an einem Auslöseelement (600), wie einer schwenkbaren Stange, befestigt ist.

5. Vorrichtung nach Anspruch 4, wobei die Sperrmittel (500) mit dem Auslöseelement (600) einstückig gebildet sind.

6. Vorrichtung nach einem der vorhergehenden Ansprüche, wobei das Behälterträgerelement (1000) mit einer Trägerplatte (1100) zusammenwirkt, die mit dem Körper (10) einstückig ausgebildet ist.

7. Vorrichtung nach Anspruch 6, wobei die Trägerplatte (1100) Halteelemente (1104) umfasst, die dazu geeignet sind, mit Halterampen (1004) des Behälterträgerelements (1000) zusammenzuwirken.

8. Vorrichtung nach Anspruch 6 oder 7, wobei die Trägerplatte (1100) geradlinige Führungsrippen (1105) umfasst, die dazu geeignet sind, mit Führungsstücken (1005) des Behälterträgerelements (1000) zusammenzuwirken.

## Claims

1. A fluid dispenser device triggered by inhaling, comprising a fluid reservoir (100) containing a fluid and a propellant gas, a metering valve (200) being assembled on said reservoir (100) for selectively dispensing the fluid, said device further comprising:
• a cocking member (800) that is movable firstly between a rest position and a primed position by compressing a spring (70), and that is axially movable secondly between said primed position and a dispensing position under the effect of the force exerted by said compressed spring;
• blocking means (500) for blocking said cocking member in said primed position; and
• a trigger system that is controlled by inhaling so as to release said blocking means (500);
the device being **characterized in that** said cocking member (800) includes a set of teeth (810), and **in that** it further comprises:
• a reservoir support element (1000) that is fastened to said reservoir (100) and that includes a cam opening (1003); and
• a meshing wheel (900) comprising firstly a toothed projection (910) that is adapted to co-operate with said set of teeth (810) of said cocking member (800), and secondly a cam projection (920) that is adapted to co-operate with said cam opening (1003) of said reservoir support element (1000), said toothed projection (910) being substantially centered relative to the axis of rotation of said meshing wheel (900), and said cam projection (920) being offset relative to said axis of rotation, said meshing wheel (900) co-operating with said cocking member (800) so that when said cocking member (800) moves from its primed position towards its dispensing position, said meshing wheel (900) performs a complete turn, such that said cam projection (920) performs a circular movement causing said reservoir support element (1000) to perform reciprocating axial movement, with a first axial movement for actuating said valve (200) and a second axial movement in the opposite direction for returning the valve into its rest position.

2. A device according to claim 1, further comprising an actuator member (50) that co-operates with said cocking member (800), said actuator member (50) being provided with a cam surface (51) for moving said cocking member (800) from a rest position towards a primed position, said actuator member (50) co-operating in said primed position with said blocking means (500) so as to hold said cocking member (800) in said primed position until inhalation, and said actuator member (50) being moved by said cocking member (800) towards a dispensing position after said blocking means (500) have been released so as to actuate said valve (200).

3. A device according to claim 2, wherein said blocking means (500) include a latch (501) that, in its blocking position, co-operates with a projection (59) of said actuator member (50).

4. A device according to any preceding claim, wherein said trigger system controlled by inhaling comprises a deformable air chamber (60) that is deformed during inhaling, said deformable air chamber (60) being fastened to a trigger element (600), such as a pivot rod.

5. A device according to claim 4, wherein said blocking means (500) are secured to said trigger element (600).

6. A device according to any preceding claim, wherein said reservoir support element (1000) co-operates with a support plate (1100) that is secured to said body (10).

7. A device according to claim 6, wherein said support plate (1100) includes holding elements (1104) that are adapted to co-operate with holding ramps (1004) of said reservoir support element (1000).

8. A device according to claim 6 or claim 7, wherein said support plate (1100) includes rectilinear guide splines (1105) that are adapted to co-operate with guide pins (1005) of said reservoir support element (1000).
